Europäisches Patentamt

(19) European Patent Office

Office européen des brevets

(11) Publication number: **0 044 818**

**A1**

(12) # EUROPEAN PATENT APPLICATION

(21) Application number: **81830080.8**

(22) Date of filing: **22.05.81**

(51) Int. Cl.³: **A 61 L 2/24**

(30) Priority: **21.07.80 IT 8340980**

(43) Date of publication of application:
. **27.01.82 Bulletin 82/4**

(84) Designated Contracting States:
**DE FR GB IT SE**

(71) Applicant: **Salvador, Pierantonio
Via IV Novembre, 20
I-33096 San Martino al Tagliamento(IT)**

(72) Inventor: **Salvador, Pierantonio
Via IV Novembre, 20
I-33096 San Martino al Tagliamento(IT)**

(74) Representative: **Petraz, Gilberto
G.L.P. S.a.s. di Gilberto Petraz P.le Cavedalis 6/2
I-33100 Udine(IT)**

(54) **Procedure for monitoring non-homogenization inside sterilization chambers, and device suitable for carrying out said procedure.**

(57) The invention concerns a procedure for monitoring non-homogenization inside sterilization chambers having an atmosphere of any desired gas wherein the sterilizing effect is obtained advantageously with steam, said procedure envisaging that the gas/steam mixture is made to pass at least partially through a device (21) in which it is cooled, whereby condensation is brought about while the temperature of the place where condensation takes place is read, and whereby the conditions inside the sterilization chamber are ascertained through the value of said temperature.

Said procedure is carried out with a device which comprises a delivery conduit (16) cooperating with a condensation chamber (18) wherein there is a measuring thermometer means (19), whereby there are cooling means (17) and there is an equalizer channel means (20) which perhaps performs, drainage, and whereby said channel means (20) and said at least partially cooled delivery conduit (16) form a closed circuit cooperating in a coordinated manner with a conduit (15) connected to said sterilization chamber (10), whose state can be indicated by optical or sonical signals.

fig.1

Croydon Printing Company Ltd.

Description of the invention entitled:

"Procedure for monitoring non-homogenization inside steril-

ization chambers, and device suitable for carrying out said

procedure"

in the name of Pierantonio SALVADOR at S. Martino al Taglia-

mento

Submitted on                                    under No.

———————————————

This invention concerns a procedure for monitoring non-homogenization inside sterilization chambers chambers. The invention also concerns a device suitable for carrying out said procedure for monitoring non-homogenization inside

sterilization chambers.

Sterilization or the process of sterilization is carried on with a plurality of operations following each other and able to accomplish the sterility of a material, whereby the sequence of said operations is called the sterilization cycle.

Said sterilization cycle synthesizes the connection between the microbiological aspects of sterilization and the technical and operating aspects of the execution of the sterilization process.

It is essential, if the cycle is to have a good outcome,

that the cycle itself should be carried out in conditions of homogeneity of the sterilizing agent in respect of the ma-

terial to be sterilized.

*Gilberto Petraz*

Industrial sterilization is most often carried out with the employment of satured steam as the sterilizing agent. It is known that with conditions of saturation it is equivalent to indicate either the temperature of the pressure so as to define the physical state of the steam; hereinafter, therefore, we shall use the two magnitudes equally to indicate said physical state.

For the sterilization cycle the saturated steam is delivered into a sterilization chamber where the material or "charge" has already been introduced for sterilization, and steam is delivered in quantities such as to saturate the enclosed atmosphere in said sterilization chamber.

A suitable relationship between the temperature of the sterilization atmosphere satured with steam and the time said saturation is maintained in said atmosphere determines the success of the sterilization cycle, that is, the sterilization of said charge. However, it is essential to carry out sterilization in a saturated atmosphere, for the presence of pockets of air inside said chamber creates conditions of insufficient sterilization or, indeed, of non-sterilization.

Said presence of air is to be found, above all, when the charge to be sterilized consists, as is usually the case, of porous materials (such as cloths or fabrics employed in hospitals or in medical circles in general) which are most likely to hold air bubbles.

Monitoring of the sterilization cycle, according to known techniques, can be carried out with a control of a biological type or with a control by physical methods of colour change.

The control of a biological type employs appropriate holders containing microbic cultures possibly in combination with other organic components.

Said holders are distributed in sufficient numbers and

Gilberto Petraz

in an even way, or according to what may be deemed to be an optimum distribution, inside the sterilization chamber and, where possible, amid the charge.

When the sterilization cycle has been completed, the holders are withdrawn and broken so that the culture broth can come into contact with the microbic culture, and the whole is kept in incubation for a given time.

Under such conditions, if there are micro-organisms still alive, they grow and reproduce themselves, thus determining in a known way a colour change in the culture broth (a thing which does not happen if alla the micro-organisms are dead). In such a case it is necessary to repeat the cycle and therefore to repeat the biological control with the employment of further cartridges or holders.

There are other methods to carry out the controls; all of them may require a possible repetition of the sterilization cycle if the results of the control are not satisfactory.

Said known procedures are especially unhelpful because of the great cost of the individual control units, the waiting times needed to know the outcome of the control and the possible necessity of repeating the cycle.

The times of said known cycles are very long, being determined by the time taken to develop the basic components which are used in the control unit and which indicate any micro-organisms possibly remaining after the sterilization, and by the time employed in checking the control results, and also by the time possibly taken up in repeating the cycle.

A further drawback of these control methods lies in the difficulty of carrying out the procedure itself.

The control of sterilization with physical colour change methods of the Bowie-Dick type, for instance, envisages the analyzer device being disposed in the middle of the charge to the sterilized and, then, to be analysed, whereby the middle of said charges forms the most critical or most dis-. advantageous position. In fact, said zone is the hardest for the saturated steam to reach and is therefore suitable for being used to accomplish a uniform control of the steriliz ation of the charge.

When control methods of the physical colour change type are employed, the Bowie-Dick analyzer is withdrawn after carrying out the sterilization cycle; if said analyzer shows a distinclty dark colouring over all its sensitive bands, it is inferred that the sterilization has a very high percentage of likelihood of success.

If said analyzer shows a light colouring in its middle bands, it is deduced that the sterilization has an unacceptable percentage of likelihood of success from the point of view of sterility of the charge, and in this event it is best to repeat the cycle and the control.

Colourings in between the foregoing have diverse like- lihoods of success.

The type of control we have just described is less accurate than the previous one but is less expensive. In this kind of control, even slight shadings of colour in the sensitive bands of the Bowie-Dick analyzer, shadings which are not appraised by the human eye, can show that in the sterilization chamber there is enough air to enable the bacteria to stay alive, thus making the sterilization ineffective.

The main purpose of our invention is to realize a control procedure which is economical, requires only a short time for analysis or verification and is extremely accurate.

The author has verified with laboratory tests the appropriateness of using the homogeneity or, contrariwise, the lack of homogeneity of the steam in the sterilization atmosphere as an effective term or reference point for an evaluation analysis of the degree of penetration of the steam into the charge.

The author himself has therefore developed a control procedure which, by using the device of the invention, makes possible a verification which is overall (and therefore not done poin by point) and immediate (in actual time) of the homogenization (or non-homegenization) of the saturated atmosphere.

The procedure we propose is based on the principle that the greater the percentage of steam in a given volume of gas, the faster said volume of gas in which the steam is present will reach saturation poin and, therefore, the beginning of condensation.

Thus, for instance, a quantity of gas which is 70% saturated with steam will reach 100% saturation point at a temperature higher than the same quantity of gas only 30% saturated with steam.

This physical principle being constant or almost constant, the content of the atmosphere within which the sterilization of the charge takes place enables us to determine, by measuring the temperature of condensation or by measuring the temperature of the atmosphere in which the condensation is caused, the degree of saturation of the atmosphere in which the sterilization takes place.

The author has therefore embodied a device suitable for the pratical application of said physical principle and which therefore enables said procedure to be carried out.

According to the invention a mixture of air and steam existing in the sterilization chamber is made to pass through a suitable device. As the less saturated the air, the colder the air-steam mixture, said mixture will tend to be placed in defined positions in the sterilization chamber. This enables a preferential path to be imposed on said mixture in the presence of a continous feed of saturated steam.

This preferential path cooperates with the device of the invention in a coordinated manner.

In said device said air-steam mixture is saturated until condensation of the steam is brough about.

Therefore, from the beginning to the end of the sterilization cycle steam forming part of a more and more saturated air-steam mixture will be condensed in the device of the invention, and this will go on until said mixture is one hundred per cent saturated.

Under such conditions the condensating temperature tends to stay constant or substantially constant.

Before reaching the constant or almost constant condensation temperature, we can find ourselves in the presence even of considerable falls of temperature when pockets of air are removed owing to the action of the saturated steam.

Other variants shown by a temperature recording may indicate that equipment is not working properly, that packings are not-tight, that slits or other defects are present in the chamber, etc., each of such shortcomings being quickly and reaily identifiable.

The greater the quantity of air inside the chamber and the greater the quantity of air in the air-steam mixture, therefore, the colder the residual air collected in the invention.

In actual fact, to small falls i temperature and therefore to small variations in the degree of non-homogenization within the sterilization chamber there correspond more significant falls in temperature which can be measured in the device of the invention.

As said earlier, such a device behaves substantially like an amplifier of falls or variations in temperature or like an amplifier of non-homogenization.

The invention, therefore, consists in a procedure for monitoring non-homogenization inside sterilization chambers. with an atmosphere of any desired gas, within which the sterilizing effect is obtained advantageously with steam, said. procedure being characterized by the fact that the gas-steam mixture is made to pass at least partially through a device. wherein it is cooled, thus obtaining its condensation, while the temperature is read of the place where the condensation. takes place, the value of said temperature being used to show the conditions inside the sterilization chamber.

Accorging to the invention the temperature can be measured continously or periodically.

According to the invention optical or analogue means can be comprised to indicate the temperature and can plotit or otherwise.

Further according to the invention the conditions inside. the sterilization chamber are translated into real-time optical or sonic alarm signals indicative of the real state of homogenization within said chamber.

Moreover, the invention is embodied in a device suitable for realizing the procedure for monitoring non-homogenization inside sterilization chambers having an atmosphere of any desired gas, wherein the sterilizing effect is bbtained advantageously with steam, and said device cooperates with the sterilization chamber and is characterized by comprising a delivery conduit cooperating with a condensation chamber wherein there is a thermometer monitoring means, whereby there are cooling means and a channel means to equalize pressure and perhaps to carry out drainage, and whereby said channel means and said at least partially cooled delivery conduit form a closed circuit cooperating in a coordinated manner with a conduit connected to said sterilization chamber.

Further according to the invention therebeing provided an external optical or sonic alarm device, connected to said monitoring means, suitable for displaying instantaneous optical indications or giving sonic signals indicative of the instantaneous state of homogenization within the sterilization chamber.

The attached tables show diagrammatically and as an example one type of non-restrictive embodiment of the device of the invention.

- Fig. 1 shows with a diagram of blocks the control (or regulation) of the sterilization cycle;

- Fig. 2 gives a graph with curves of the temperatures expressed as a function of time and measured inside the sterilization chamber, and with curves of the temperatures expressed once again ad a funcion of time and measured as responses at the output of the device;

- Fig. 3 shows a true development of the responses as a function of time in correspondence with diverse values of loss of pressure inside the sterilization chamber;

- Fig. 4 gives the response in time during the actual sterilization phase itself;

- Fig. 5 shows a view of a section of the device employing the physical principle to which the invention refers.

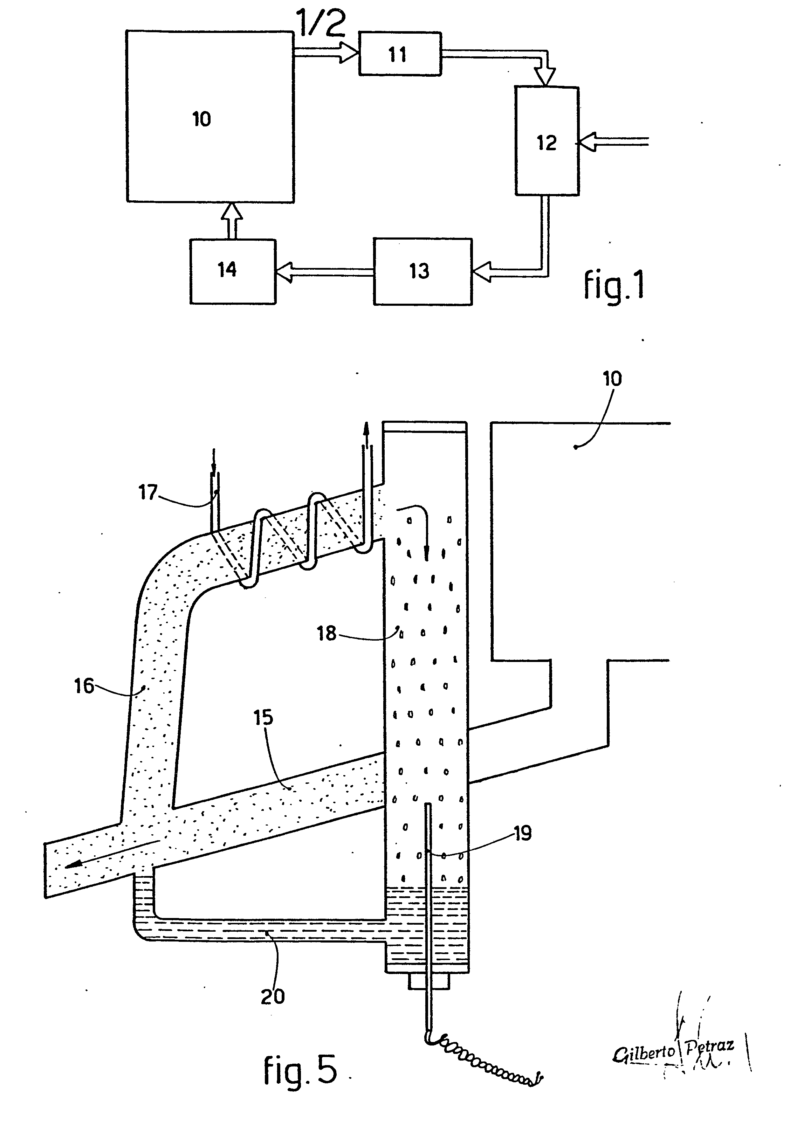

In Fig. 1 is shown the diagram of blocks of the control and regualtion of the sterilization cycle.

Actual sterilization takes place inside the sterilization chamber 10 and is monitored in real time by the transducer 11 consisting of the device adopting the invention.

The output signal from the transducer is compared in a monitoring and/or regulating unit 12 with signals of a preset value.

Gilberto Petraz

Said unit sends out a command signal, which is suitably converted by the transducer 13 and serves to pilot the actuation organs 14 able to generate conditions of sterilization inside the chamber 10.

The flow lines connecting the various blocks show the preferential path followed by the control signals.

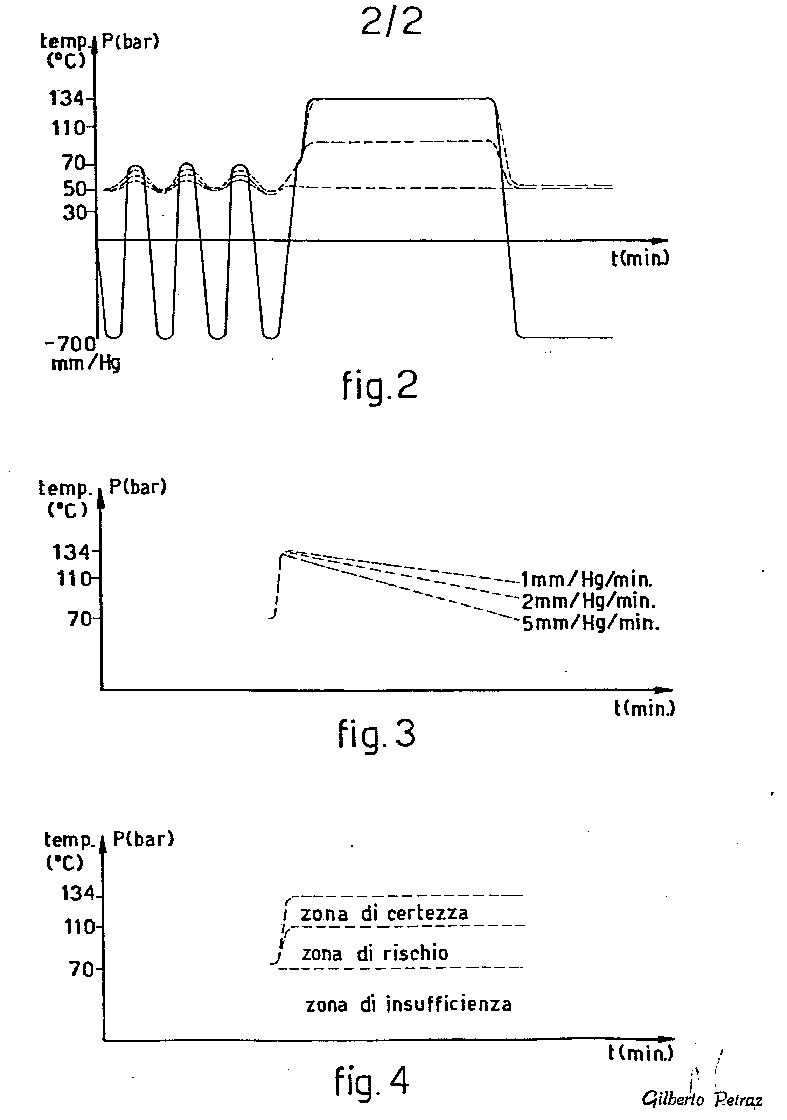

Fig. 2 shows graphs of the temperatures, as a function of time, of the steam inside the sterilization chamber and of the residual air inside the device of the invention.

The variations in temperature or, as we said earlier, the variations in pressure of the saturated steam inside the chamber are shown with continuous lines in Fig. 2. In particular the sterilization process envisages a preliminary phase of empting and charging the chamber 10 with saturated steam so as to obtain thereafter better filling of the said chamber 10 with steam. This phase is shown in Fig. 2 with the initial tract having an impulsive form.

Saturated steam is the injected once more into the chamber 10 and the temperature (and pressure) of said steam is increased until it reaches the optimum value known for sterilization with satured steam; this value can be, for instance, 134°C. This temperature is mainteined for the whole of the sterilization phase, at the end of which conditions of vacuum are created so as to dry the charge in the chamber 10.

Said conditions of vacuum are shown in the end tract of the graph with a pressure value of minus 700 mm. Hg.

The graphs of the control signals given with lines of dashes in Fig. 2 are obtained by using the device 21 shown diagrammatically in Fig. 5. Inside the device 21 the steam discharged from the chamber 10 passes through the discharge pipe 15 and penetrates into the delivery conduit or condensate pipe 16.

The cooling water which runs through the cooling coil 17 causes said steam to condense in the condensation chamber or air-collection pipe 18, or in 16, and said condensed steam runs in the form of drops into the equalizer channel or compensating pipe 20.

Any air present in the steam will then be pushed by the flow of steam coming from the sterilization chamber towards the higher part of the condensate pipe 16 and then into the air-collection pipe 18.

As air is denser than steam, it will flow to the bottom of the air-collecting pipe 18, where it will be concentrated, for its re-circulation in hindered by the water present in the compensating pipe 20.

In said compensating pipe 20 a monitoring thermometer 19, of a thermocouple type for instance, is disposed above the water level and measures the variations in temperature, converting them in this example into variations of tension and then into electric signals which can be taken bu a suitable output outside said compensating pipe 20.

The greater the quantity of air in the steam inside the chamber 10, the greater the drop in temperature which can be measured with the thermocouple 19, or the greater the non-homogenization of the steam inside the chamber 10, the greater the variation in the output signal of the thermocouple 19.

In actual fact, greater output signals correspond to small variations in homogeneity, so that, as we said earlier, the device of the invention behaves like an amplifier of non-homogenization.

Going back to the sterilization cycle of Fig. 2, we can see that three responses have been plotted with regard to as many inputs, said responses having been conditioned by differing degrees of non-homogenization within the chamber 10.

*Gilberto Petraz*

Let us now loog at a non-restrictive example referring to specific losses of pressure. Let us consider a loss of pressure of one to two mm. Hg per minete during the phase of vacuum in the chamber 10.

In the first tract of the graph the response tends, with a certain inertia, to follow the temperature pulsations (or pressure pulsations) imposed on the steam, whereby said oscillations, however, have a minimum value of temperature which can be estimated experimentally to be about 50°C.

In the middle part of the graph relating to the actual sterilization period itself the response follows the variations in the input magnitude almost faithfully and with an inertia which can be overlooked.

The end part of the cycle is not relevant for the purposes of the invention and is therefore not taken into account by us now or hereinafter.

If there is a pressure loss of 2 to 3 mm. Hg per minute in the chamber during the vacuum phase, the response given by the invention follows the variations in the input magnitudes with a greater inertia. Said response in the part of the graph concerned has oscillations with very modest peacks as compared to the input, as shown in Fig. 2; in the middle part the response is stabilized at a value which can be comprised between about 110° and 75°C.

In the third condition with losses greater than 3 mm. Hg per minute the response tends to be kept almost constant at about a minimum value of 70°C (value of minimum threshold).

These responses enable us to pick out three distinct control zones (Fig. 4).

If the temperature inside the chamber is 134°C and the loss of pressure therein is not more than 2 mm. Hg. per minute, the invention indicates a temperature of about 134°C.

It has been ascertained in the author's laboratories that said response indicates safe sterilization.

If there is a loss of pressure of 2 to 3 mm. Hg per minute within the chamber at a temperature of 134°C, the device of the invention measures a temperature defined within a range of values which indicate a zone of risk owing to the uncertainity of success of the sterilization.

[ If there is a loss of pressure greater than 3 mm. Hg per minute within the chamber at a temperature of 134°C, the device of the invention indicates a temperature definied below the minimum value; in this case the sterilization has not succeded.

The invention, therefore, makes it possible not only to amplify a signal correlated, as said earlier, to non-homogenization whithin the chamber 10, but also to make clear, in real time, the result of the process monitoring the sterilization by discriminating between three kinds of responses which, depending on the results obtained in output, and in relation to the three aforesaid ranges of values, are: successful sterilization, uncertain sterilization and unsuccessful sterilization.

In fact to enhance the invention's real-time monitoring potential and its utility it is proposed to provide the device according the invention with an alarm device connected directly to the output of the monitoring and/or regulating unit 12.

Said alarm device may be a visual display capable of switching on one  of three differently coloured indicator lights each indicator right being associated with one of the above mentioned working conditions.

Namely, a green light for successful sterilization; a yellow light for uncertain sterilization and red light for an unsuccsseful sterilization.

Gilberto Petraz

The said alarm device may on the other hand be a visual display on which said conditions are indicated in writing.

On further type of alarm that can be used is a sonic alarm device which emits three types of audible signals whose respective frequencies or amplitudes correspond each to one of the three states of sterilization mentioned hereinabove.

The sonic alarm device may be activated manually or may go into action only when it is necessary to warn the operator.

Following on such considerations, we can define the device of the invention as a discriminating amplifier of non-homogenization.

Fig. 3 shows the graphical development of the true response of the device 21 with reference to the aforesaid non-restrictive example, corresponding to the zone of the beginning of sterilization. With said graph we can ascertain whether during the beginning phase (said phase of homogenization) there are in the chamber such losses as to cause the cycle for obtaining sterilization to be halted.

If said graph shows a starting peak of an amplitude which can be estimated to be about 134°C, this being the temperature used for the sterilization phase, as we said earlier, this means that the chamber 10 is in a condition to perform sterilization properly.

If said peak has an amplitude positioned in the zone of risk or corresponding to minimum values or to values the same as the minimum, it is best to check and put right any losses of pressure in the chamber 10 or any introduction of steam containing too much air.

The diverse slopes of the true curves shown in Fig. 3, obtained at the output of the device of Fig. 5 and estimated for various losses of pressure in the chamber 10, being expressed in mm. Hg. per minute and referring to a pre-set volume V of the chamber 10, are due to the fact that the steam introduced into the chamber 10 during sterilization always contains some parts of air in actual fact.

Therefore, the device 21 deduced by the invention enables the degree of non-homogeneity, as defined earlier, to be kept under continual or periodical surveillance (control, monitoring) even during the whole phase of sterilization.

We have described here a procedure and a device 21 able to embody said procedure, but variants are possible.

Thus the proportions, values and sizes can be varied and parts can be added or integrated; the non-essential shapes can pe varied and the conduit 16 can have any desired shape; the conduit 16 can be at least partially insulated; cooling 17 can take place in the chamber 18 and can be obtained in any desired manner, etc. These and other variants are all possible for a technician in this field.

*Gilberto Petraz*

0044818

rif. glp 3-2692.nc

## C L A I M S

1. Procedure for monitoring non-homogenization inside sterilization chambers having an atmosphere of any desired gas wherein the sterilizing effect is obtained advantageously with steam, charcaterized by the fact that the gas/ steam mixture is made to pass at least partially through a device (21) in which it is cooled, whereby condensation is obtained while the temperature of the place where condensation occurs is measured, and whereby the conditions inside the sterilization chamber are ascertained through the value of said temperature.

2. Procedure for monitoring non-homogenization inside sterilization chambers, as in claim 1, characterized by the fact that measurement of the temperature takes place continuously.

3. Procedure for monitoring non-homogenization inside sterilization chambers, as in claim 1, characterized by the fact that measurement of the temperature takes place at preferential periods.

4. Device to carry out the procedure for monitoring non-homogenization inside sterilization chambers (10) having an atmosphere of any desired gas wherein the sterilizing effect is obtained advantageously with steam, whereby said device (21) cooperates with the sterilization chamber (10), said device being characterized by comprising a delivery conduit (16) cooperating with a condensation chamber (18) in which there is a measuring thermometer means (19), whereby there are cooling means (17) and there is an equalizer channel means (20) which perhaps performs drainage, and whereby said channel means (20) and said at least partially cooled delivery conduit (16) form a closed circuit cooperating in a coordinated manner with a conduit (15) connected to said sterilization chamber (10).

*Gilberto Petraz*

5. Device to carry out the procedure for monitoring non-homogenization inside sterilization chambers (10), as in claim 4, characterized by the fact that the measuring thermometer means (19) stretches into the condensation chamber (18) above the level of the condensate.

6. Device to carry out the procedure for monitoring non-homogenization inside sterilization chambers (10), as in claim 4 and 5, characterized by the fact that the measuring thermometer means (19) can be connected to any desired means performing reading, monitoring, transcription, control or processing.

7. Device to carry out the procedure for monitoring non-homogenization inside sterilization chambers (10), as in claim 4 and in claim 5 or 6, characterized by the fact that the cooling means (17) cooperate at least partially with the delivery conduit (16).

8. Device to carry out the procedure for monitoring non-homogenization inside sterilization chambers (10), as in claim 4 and in claim 5 or 6, characterized by the fact that the cooling means (17) cooperate at least partially with the condensation chamber (18).

9. Procedure for monitoring non-homogenization inside sterilization chambers as in claim 1 or one or the other up to and inclusive of claim 3, characterized by the fact that said conditions inside the sterilization chambers are indicated by corresponding external warning optical or sonic signals.

10. Device to carry out the procedure for monitoring non-homogenization inside sterilization chambers (10) as in any of claims 4 to 8 inclusive, characterized by the fact that said device includes an optical display alarm device emitting distinct optical signals indicative of the corresponding sterilization conditions inside said chambers.

11. Device to carry out the procedure for monitoring non-homogenization inside sterilization chambers (10) as in any of claims 4 to 8 inclusive characterized by the fact that said device includes an optical alrm device capable of indicating in writing the instantaneous state of sterilization within said chambers.

12. Device to carry out the procedure for monitoring non-homogenization inside sterilization chambers (10) as in any of claims 4 to 8, characterized by the fact that said device includes a sonic alarm device emitting distinct audible signals whose respective frequencies or amplitudes correspond to the instantaneous state of sterilization within said chambers, whereby said sonic alarm device emits said audible signals interminably or intermittently.

13. Procedure for monitoring non-homogenization inside sterilization chambers (10) having an atmosphere of any desired gas wherein the sterilizing effect is obtained advantageously with steam, and device to carry out the procedure for monitoring non-homogenization inside sterilization chambers (10) having an atmosphere of any desired gas wherein the sterilizing effect is obtained advantageously with steam, whereby said device (21) cooperates with the sterilization chamber (10), as in one or another of the claims hereinbefore, and as described and shown and for the purposes allowed.

fig.1

fig.5

Gilberto Petraz

fig.2

fig.3

fig.4

Gilberto Petraz

0044818

European Patent Office

**EUROPEAN SEARCH REPORT**

Application number

EP 81 83 0080

| | DOCUMENTS CONSIDERED TO BE RELEVANT | | CLASSIFICATION OF THE APPLICATION (Int Cl.³) |
|---|---|---|---|
| Category | Citation of document with indication, where appropriate, of relevant passages | Relevant to claim | |
| | <u>GB - A - 1 169 179</u> (MANLOVE) <br> * Claims; figures 1-3 * <br><br> -- | 1-13 | A 61 L 2/24 |
| A | <u>US - A - 3 454 353</u> (B.A. BJORK) <br> * Claims * <br><br> ---- | 1 | |

| TECHNICAL FIELDS SEARCHED (Int. Cl.³) |
|---|
| A 61 L 2/24 |

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 27-10-1981 | MERGONI |

EPO Form 1503 1 06.78